(19) Europäisches Patentamt
European Patent Office
Office européen des brevets

(11) Publication number: **0 169 169**
**B1**

(12) **EUROPEAN PATENT SPECIFICATION**

(45) Date of publication of patent specification: **17.10.90**

(21) Application number: **85810310.4**

(22) Date of filing: **05.07.85**

(51) Int. Cl.[5]: **C 07 C 333/04,**
C 07 C 275/00, C 07 C 335/04,
C 07 C 69/96, C 07 C 329/02,
C 07 C 321/22, A 01 N 47/12,
A 01 N 47/14, A 01 N 47/28,
A 01 N 47/06

# (54) Novel aromatic compounds.

(30) Priority: **18.07.84 US 631959**

(43) Date of publication of application:
**22.01.86 Bulletin 86/04**

(45) Publication of the grant of the patent:
**17.10.90 Bulletin 90/42**

(84) Designated Contracting States:
**AT BE CH DE FR GB IT LI NL**

(56) References cited:
**DE-A-2 150 956**
**FR-A-1 584 844**
**GB-A-1 259 472**
**GB-A-1 382 204**
**US-A-3 277 142**
**US-A-4 451 474**

(73) Proprietor: **SANDOZ AG**
**Lichtstrasse 35**
**CH-4002 Basel (CH)**
(84) **BE CH FR GB IT LI NL**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN**
**Verwaltungsgesellschaft m.b.H.**
**Brunner Strasse 59**
**A-1235 Wien (AT)**
(84) **AT**

(72) Inventor: **Henrick, Clive Arthur**
**3177 Manchester Ct.**
**Palo Alto, CA 94303 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 169 169 B1

## Description

The present invention relates to novel aromatic compounds for the control of pests. Though many pest control agents such as the IGR compounds methoprene, hydroprene and fenoxycarb are known, the need exists for more effective IGR compounds (e.g. more active compounds) and/or compounds having different spectrum of activity).

This invention provides novel compounds of the formula I

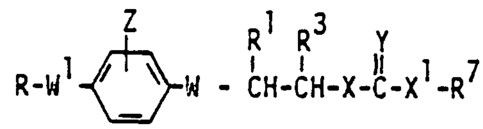

I

wherein

R is $C_{1-8}$alkyl, $C_{2-8}$alkenyl, $C_{2-8}$alkynyl, $C_{1-8}$haloalkyl, $C_{2-8}$haloalkenyl, $C_{2-8}$haloalkynyl, $C_{2-10}$alkoxyalkyl, $C_{2-10}$alkylthioalkyl, $C_{3-8}$cycloalkyl, $C_{3-8}$halocycloalkyl, $C_{4-12}$cycloalkylalkyl, heterocycloalkyl or heterocycloalkylalkyl;

either of

$R^1$ or $R^3$ is hydrogen or $C_{1-5}$alkyl, and the other is hydrogen;
$R^7$ is $C_{1-5}$alkyl, $C_{3-6}$cycloalkyl or $C_{3-5}$alkenyl;
W is oxygen or sulfur;
$W^1$ is oxygen, sulfur or carbonyl;
X and $X^1$ independently are oxygen, sulfur, NH or N($C_{1-5}$alkyl);
Y is oxygen or sulfur; and
Z is hydrogen, $C_{1-8}$alkyl, $C_{1-8}$haloalkyl or halogen.

The compounds of formula I have interesting pest controlling, in particular insect controlling properties.

The compounds of the present invention of formula I can be prepared by methods known in the art, such as those described in U.S. Patents 4,080,470 and 4,215,139, European Patent 98,800 for example, and as outlined below.

A convenient method involves O, S or N-alkylating or -acylating an amino-, alkoxy, phenoxy or thio-moiety of the compound of formula I with the other moiety of the compounds of formula I to form a (thio)ether, (thio)ester, amine or (thio)amide. Such method can be carried out under conditions known for the preparation of (thio)ethers, (thio)esters, amines or (thio)amides starting from the corresponding alcohols, phenols, thiols and amines using known O-, S- or N-alkylating or O-, S- or N-acylating techniques for the preparation of (thio)ethers, amines, (thio)carbamates, (thio)urea and (thio)carbonates. Suitable O, S- or N-alkylating resp. O-, S- or N-acylating agents are halides (e.g. alkyl- or acylhalides) of formulae III, VI, VIII, IX or X hereinafter or reactive functional derivatives thereof such as mesylate (e.g. of formula VIII), alkali metal salts of (thio)carboxylic acids (e.g. of formula IX) and iso(thio)cyanates (e.g. of formula IV).

Suitable examples of such method for preparing compounds of formula I comprise

a) reacting a compound of formula II

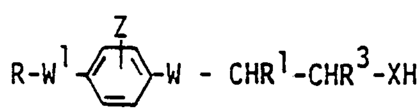

II

wherein R, $W^1$, Z, W, $R^1$, $R^3$, and X are as defined above, with a compound of formula III

$$Q—CY—(X^1)—R^7 \qquad \text{III}$$

wherein Y, $X^1$ and $R^7$ are as defined above, and Q is halogen or

b) obtaining a compound of formula Ia

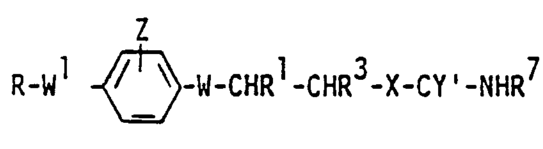

Ia

wherein R, $W^1$, Z, W, $R^1$, $R^3$, X and $R^7$ are as defined above, and Y′ is O or S, by reacting a compound of formula II, as defined above, with a compound of formula IV

$$Y'=C=NR^7 \qquad \text{IV}$$

wherein Y′ and $R^7$ are as defined above,

c) obtaining a compound of formula Ib

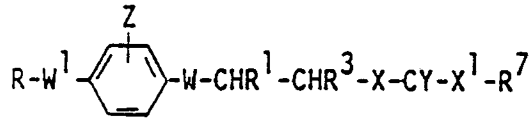

Ib

wherein W, R, $W^1$, Z, $R^1$, $R^3$, X, Y, $X^1$ and $R^7$ are as defined above, by reacting a compound of formula V

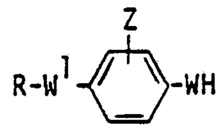

V

wherein R, $W^1$, Z and W are as defined above, with a compound of formula VI

$$Q—CHR^1—CHR^3—X—CY—(X^1)—R^7 \qquad VI$$

wherein Q, $R^1$, $R^3$, X, Y, $X^1$ and $R^7$ are as defined above,

d) obtaining a compound of formula Ic

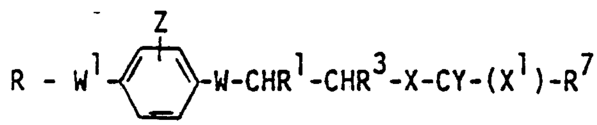

Ic

wherein $W^1$, Z, W, $R^1$, $R^3$, X, Y, $X^1$, R and $R^7$ are as defined above, by reacting a compound of formula VII

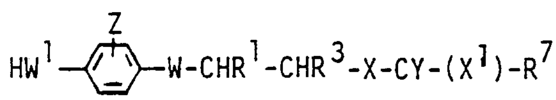

VII

wherein $W^1$, Z, W, $R^1$, $R^3$, X, Y, $X^1$ and $R^7$ are as defined above, with a compound of formula VIII

$$RQ^1 \qquad VIII$$

wherein R is as defined above, and $Q^1$ is halogen or mesyl,

e) obtaining a compound of formula Id

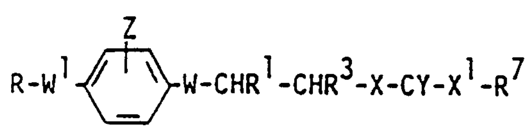

Id

wherein R, $W^1$, Z, W, $R^1$, $R^3$, X, Y, $X^1$ and $R^7$ are as defined above, by reacting a compound of formula IX

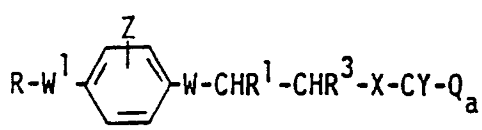

IX

wherein

$Q_a$ is OM, SM or halogen,

M is an alkali metal,

R, $W^1$, Z, W, $R^1$, $R^3$, X, and Y are as defined above, with a compound of formula X

$$Q_bR^7 \qquad X$$

wherein $Q_b$ is either halogen, where $Q_a$ is OM or SM, or is OM or SM where $Q_a$ is halogen, and $R^7$ is as defined above.

The above reactions are known per se; they may be effected under conditions known to a person

skilled in the art. They are conveniently carried out in an organic solvent which is inert under the reaction conditions, such as N-methylpyrrolidone, dimethylformamide or tetrahydrofuran.

A suitable reaction temperature is between −5° and 140°, e.g. 10° and 110°.

Where one reaction partner is an halide, it is in general preferred to effect the reaction in the presence of a base such as $K_2CO_3$ or NaOH, or to react the halide with a salt form (e.g. Na salt) of the alcohol, phenol, phenylthiol or amine or (thio)carboxylic acid.

The compounds of formula I may be recovered from the reaction mixture in which they are formed by working up by established procedures.

The compounds of the present invention of formula I can have one or more asymmetric carbon atoms. The present invention includes each of the optical isomers and racemic mixtures thereof. In the examples hereinafter, unless otherwise specified, the compound prepared is a racemic mixture.

The starting materials and reagents employed in the processes described above are either known or, insofar as they are not known, may be produced in analogous manner to the processes described herein or to known processes.

Where R is $C_{1-8}$alkyl, $C_{2-8}$alkenyl or $C_{2-8}$alkynyl, it refers particularly to an alkyl, alkenyl or alkynyl group having 2—6, more preferably 4 or 5 carbon atoms; such groups are preferably branched. Particularly preferred alkyl significances of R are 2-methyl-butyl and, especially secondary butyl. A particularly preferred alkenyl significance of R is 3-methyl-2-butenyl (i.e. $CH_3C(CH_3)=CHCH_2$).

The terms $C_{1-8}$haloalkyl, $C_{2-8}$haloalkenyl, $C_{2-8}$haloalkynyl, $C_{3-8}$halocycloalkyl and $C_{1-8}$haloalkoxy refer to such hydrocarbyl groups substituted by 1 to 6, particularly 1 to 3 halogen atoms. The halogen is preferably Cl or F.

The terms alkenyl and alkynyl when used herein refer to such hydrocarbyl groups having 1 or 2, preferably 1, ethylenic bonds or 1 or 2, preferably 1, acetylenic bonds resp.

The term "heterocycloalkyl" refers to a heterocycloalkyl group, saturated or unsaturated, of two to six carbon atoms and one to three atoms selected from nitrogen, oxygen or sulfur. The term "heterocycloalkylalkyl" refers to a heterocycloalkyl group wherein one hydrogen is replaced by a lower alkyl group, the total number of carbon atoms being from three to twelve.

Preferred compounds of formula I have 1 or more of the following features:

R is preferably $C_{1-8}$alkyl or $C_{2-8}$alkenyl, more preferably $C_{1-8}$alkyl.

$W^1$ is preferably O or S.

Z is preferably H, halogen, $CH_3$ or $CF_3$, more preferably H.

W is preferably O.

Where $R^1$ or $R^3$ is $C_{1-8}$alkyl it is preferably $C_{1-5}$alkyl, more preferably $CH_3$.

X is preferably O or NH.

$X^1$ is preferably S, O, NH or N($C_{1-2}$alkyl).

$R^7$ is preferably $C_{1-5}$alkyl, e.g. $CH_3$ or $C_2H_5$.

Y is preferably O or S.

A preferred subgroup of compounds of formula I, comprises the carbamates and thiocarbamates of formula I (i.e. compounds of formula I wherein either of X and $X^1$ is O or S and the other is NH— if $X^1$ is O or S — or NH or N($C_{1-5}$alkyl) — if X is O or S; hereinafter compounds of formula If). The above specified preferences apply self-evidently also for this subgroup of the formula If. Where X is S or O, $X^1$ is preferably NH or N($C_{1-5}$alkyl). Where $X^1$ is O or S, X is preferably NH.

Application of a compound of formula I is made according to conventional procedures, involving the application to the pest (insects, mites, ticks) or a locus of the pest, a pest controlling amount of a compound of formula I.

The optimum usage of a compound of formula I is readily determined by a person of ordinary skill in the art using routine laboratory testing. Usually satisfactory results are obtained with a test amount of the order of 0.1 μg to 100 μg per insect, mite or tick, depending on the mode and conditions of application as well as on the pest involved.

The compounds of formula I show interesting activity against a wide range of pests, such as fleas (Ctenocephalides felis), ticks, flies (Musca domestica), cockroaches, Blattella germanica, eggs of Spodoptera species etc. In view of their interesting activity the compounds can be effective control agents for insects of, for example, the orders Lepidoptera, Hemiptera, Homoptera, Coleoptera, Diptera, Orthoptera, and Siphonaptera, and other insects, mites and ticks of the class Acari, including mites of the families Tetranychidae or Tarsonemidae and ticks of the families Argasidae and Ixodidae.

They are preferably applied to the immature insect, namely during the egg, embryo, larval prepupal stage, in view of their effect on metamorphosis and otherwise abnormal development leading to death or inability to reproduce. For many of the compounds of formula I the effective application rate is equivalent to or below that known for commercially available IGR compounds such as methoprene, hydroprene etc. In view of their interesting pest controlling effect the compounds of formula I are indicated for use in i.a. crop protection, forest protection, stored grain protection, cattle and pet protection, against cockroaches etc.

Examples of especially useful compounds are the compounds A-19, A-21, A-39, A-88, B-7, B-26, B-31, B-34 and particularly the compounds A-1, A-13, A-14, A-30, A-31, A-36, A-38, A-40, A-82, A-83, A-86, B-1, B-7, B-10, B-12, B-15, B-17, B-23, B-27, B-30, B-31 and C-10 (of Tables A, B and C hereinafter).

The compounds of formula I are conveniently employed in pest controlling composition form in association with a diluent.

Such compositions also form part of the present invention. They may contain, aside from a compound of formula I as active agent, other active agents, such as insecticides (e.g. synthetic pyrethroids, carbamates, phosphates), insect growth regulators or insect attractants. They may be employed in either solid or liquid forms e.g. in the form of a wettable powder or an emulsifiable concentrate incorporating conventional diluents. Such compositions may be produced in conventional manner, e.g. by mixing the active ingredient with a diluent and optionally other formulating ingredients such as surfactants.

The term diluents as used herein means any liquid or solid agriculturally acceptable material which may be added to the active constituent to bring it in an easier or improved applicable form, respectively to a usable or desirable strength of activity. It can for example be talc, kaolin, diatomaceous earth, xylene, or water.

Particularly formulations to be applied in spraying forms such as water dispersible concentrates or wettable powders may contain surfactants such as wetting and dispersing agents, e.g. the condensation product of formaldehyde with naphthalene sulphonate, an alkylarylsulphonate, a lignin sulphonate, a fatty alkyl sulphate, an ethoxylated alkylphenol and an ethoxylated fatty alcohol.

In general, the formulations include from 0.01 to 90% by weight of active agent from 0 to 20% by weight of agriculturally acceptable surfactant and 99.99 to 10% by weight (solid or liquid) diluent(s), the active agent consisting either of at least one compound of formula I or mixtures thereof with other active agents. Concentrate forms of compositions generally contain between about 2 and 90%, preferably between about 5 and 65% by weight of active agent. Application forms of formulations may for example contain from 0.01 to 25% by weight, preferably from 0.01 to 5% by weight of active agent or more diluted forms thereof: they include sprays, foggers, baits, encapsulated form, cyclodextrin inclusion complexes, insect disks, pet collars, ear tags, insect bolus and the like.

The following examples are provided to illustrate the practice of the present invention. Temperatures are given in degrees centigrade and parts and percentages are by weight. RT means room temperature.

## Composition Example

Emulsifiable Concentrate

65 Parts of a compound of formula I, e.g. compound A-1 hereinafter given, are mixed with 8 parts of an emulsifier (e.g. 4 parts of Atlox 3404 F and 4 parts of Atlox 848, which are mixtures of anionic and non-ionic emulsifiers of ICI America) and 27 parts of an organic solvent (e.g. xylene or Tenneco 500-100, which is a mixture of trimethyl benzene and xylene solvents of Tenneco Corporation) are thoroughly mixed until a homogeneous solution is obtained.

## Example 1

Ethyl N-{2-[4-(3-methyl-2-butenoxy)phenoxy]-1-methylethyl}carbamate (process a))

To 2-[4-(3-methyl-2-butenoxy)phenoxy]-1-methylethylamine (0.86 g, 3.7 mmol) and pyridine (0.64 g, 8.0 mmol) in 8 ml of ether at 5° is added dropwise over 10 minutes ethyl chloroformate (0.43 g, 4.0 mmol) in 2 ml of ether. The mixture is stirred at 5° for 1 hour and is then allowed to warm to RT. Excess ethyl chloroformate is quenched with water and the reaction mixture is poured into water and ether and the aqueous phase is extracted with ether (3×). The combined organic layers are washed with 2N ammonium sulfate, with 10% sodium carbonate, then with water until neutral and with brine. The organic layer is dried, filtered, the solvent removed and the product purified by prep. TLC to give the title compound; m/s ($M^+$) 307 (Compound C-1 Table C).

## Example 2

O-2-[4-(1-methylpropoxy)phenoxy]-ethyl N-ethylthiocarbamate (process b))

To sodium hydride (0.15 g, 6.0 mmol), prewashed with pentane, in 5 ml of DMF and cooled in an ice bath, is added 2-[4-(1-methylpropoxy)phenoxy]ethanol (1.26 g, 6.0 mmol) in 5 ml of DMF. The mixture is stirred for 20 minutes at ca. 5°, after which it is heated to 50° for 20 minutes. The mixture is again cooled to ca. 5° and ethyl isothiocyanate (0.58 ml, 6.0 mmol) and 5 ml DMF are added. After 1 hour at 5°, the mixture is allowed to warm to RT, and any residual sodium hydride is destroyed by addition of 2 drops of methanol. The reaction mixture is poured into water and acidified with 3*N* sulfuric acid. The aqueous phase is extracted with ether (3×), and the combined organic phases are washed with water, with 10% sodium bicarbonate, with water and with brine, dried and filtered and the filtrate is concentrated *in vacuo* to give, after purification with prep. TLC, the title compound (compound A-36, Table A).

## Example 3

Ethyl N-{2-[4-(3-methyl-2-butenoxy)phenoxy]ethyl}-carbamate (process c))

A mixture of 4-(3-methyl-2-butenoxy)phenol (1.64 g, 9.2 mmol), ethyl 2-chloroethylcarbamate (1.81 g, 11.9 mmol), and potassium carbonate (2.54 g, 18.4 mmol) in 20 ml of dimethylformamide (DMF) is heated at 85° for 18 hours. The reaction mixture is cooled to room temperature (RT), poured into water and extracted with ether. The combined ether extracts are washed with water until neutral followed with brine and dried over calcium sulfate. The solvent was removed *in vacuo* and the residue was placed under high

vacuum at 80° for ca. 2 hours to remove the excess ethyl 2-chloroethylcarbamate. Purification by preparative thin layer chromatography (prep. TLC) gave the title compound (compound A-1, Table A).

nmr $CDCl_3$ = 1.23 (t, J=6Hz, 3H, —$OCH_2CH_3$), 1.77 (m, 6H, vinyl methyl), centered at 3.5 (m, 2H, methylene adjacent to NH), 4.03 (m, 4H, methylene adjacent to O), 4.39 (d, J=7Hz, 2H, vinyl methylene), centered at 5.25 (bm, 2H, NH and vinyl proton) and 6.8 (s, 4H, aromatic protons) ppm.

Example 4

Ethyl N-{2-[4-(1-methylpropoxy)phenoxy]ethyl}carbamate (process d))

To pre-washed sodium hydride (0.106 g, 4.4 mmol) in 5 ml of tetrahydrofuran (THF) and 5 ml of DMF at RT is added ethyl *N*-(2-(4-hydroxyphenoxy]ethyl)carbamate (1.0 g, 4.4 mmol) in 5 ml of THF. The mixture is stirred at RT for 1.5 hours and is then cooled to −5°. 1-Methylpropyl bromide (0.72 g, 5.3 mmol) in 2 ml of THF is added to the mixture, followed by addition of 5 ml of DMF. The reaction mixture is allowed to warm slowly to RT, and is then heated at 60° for 18 hours. The reaction is cooled to RT and poured into water and the mixture is extracted with ether (3×). The combined ether layers are washed with water until neutral, followed with brine, and dried over calcium sulfate. The solvent is removed *in vacuo* and purification by prep. TLC yields the title compound (compound A-13, Table A).

nmr ($CDCl_3$) = centered at 0.93 (m, 3H, $CH_3CH_2CH(CH_3)$—O], centered at 1.23 (t and d, 6H, $CH_3CH_2CH(CH_3)$—O— and $OCH_2CH_3$], centered at 1.56 [m, 2H, $CH_3CH_2CH(CH_3)$—O—], centered at 3.47 (m, 2H, methylene adjacent to NH—), centered at 4.03 (m, 5H, methylene and methine adjacent to —O—), centered at 5.07 (bm, 1, NH), and 6.77 (s, 4H, aromatic protons) ppm.

Example 5

S-Ethyl N-{2-[4-(1-methylpropylthio)phenylthio]-ethyl}dithiocarbamate (process e)

To the potassium salt of *N*-[4-(1-methylpropylthio)phenylthioethyl]dithiocarbamic acid (10.0 mmol) dissolved in 30 ml of DMF is added iodoethane (1 ml, 12.5 mmol), under $N_2$ and with stirring. The mixture is stirred at RT overnight and is then worked up by addition of water and extraction with ether. The ether layer is washed with 10% sulfuric acid, with water and with brine, dried, the solvent removed and the product is purified by column chromatography to give the title compound A-68, Table A).

Analogous to the processes a, b, c, d and e, of the examples 1 to 5, and within the limitations immanent to the process used (see description), the following compounds of formula I, Ia, Ib, Ic and/or Id listed in Tables A, B and C hereinafter are obtained.

Test Examples

Example T-1

The compounds A-12, A-13, A-30 and A-36 are tested for contact activity on houseflies by the following method.

Third instar, post-feeding wandering *Musca domestica* L. larvae are individually treated topically with 1 µl of the test compound in acetone at different dose rates. Additional larvae are treated identically with 1 µl of acetone as the control. Larvae are held in covered containers for 7 days at 31° and 16 hour photoperiod. The assay effect is expressed as $ED_{50}$, which is the dose, in µg per larva, required to cause an effect in 50% of the test insects. Effects observed include direct toxicity (larval death); delayed toxicity (pupal death); and juvenile hormone activity, such as failure of adults to emerge completely, chitin inhibition, destortion of cuticle and pupation abnormalities. Each of the above tested compounds had an $ED_{50}$ of >0.030 µg/larva.

Example T-2

The compounds A-1, A-30, A-38 and A-39 are tested for activity on the yellow fever mosquito as follows.

Late fourth instar *Aedes aegypti* larvae (generally 5 days post-hatching) are placed in plastic containers with 50 ml tap water into which has been mixed 50 µl of acetone dilution of the test compound at the concentration to be tested. A few drops of a liver powder suspension are added as a food source. The containers are covered and held at 28°, 16 hour photoperiod until all larvae or pupae are either dead or have emerged as adults. The assay effect is expressed as $EC_{50}$, which is the concentration, in ppm, required to cause an effect in 50% of the test insects. Effects observed include direct toxicity (larval death) and juvenile hormone activity such as pupal mortality and failure of adults to emerge completely. Each of the above tested compounds had an $EC_{50}$ of <0.0010 ppm.

Example T-3

Activity of the compounds A-13, A-38 and A-39 when ingested by the German cockroach are tested as follows.

Treated food is prepared by mixing the test compound into dog food at the dosage rates to be tested. One hundred 3rd instar *Blatella germanica* nymphs are placed in a cage. Water and harborage are provided and a constant supply of the treated dog food is maintained. A control with untreated dog food is also run. The cages are kept at 28°, 16 hour photoperiod and 50% relative humidity. The roaches are observed for the development of normal or abnormal adults. Normal adults are those which have fully developed wings of

the normal flat shape. Also observed is the percent control of reproduction. At 10 ppm, each of the above compounds gave 100% control of reproduction.

### Example T-4

Activity of the compounds A-1 and A-13 on the cat flea are tested as follows.

The inside bottoms of glass petri dishes (20 mm × 100 mm dishes) are treated with 1 ml of an acetone dilution of the test compound at the concentration to be tested. Approximately hone hour after treatment, ⅛ teaspoon of flea rearing medium consisting of 50% sterilized sand and 50% finely ground flea food, is placed into each dish. Five one-week-old final instar larvae of cat fleas, *Ctenocephalides felis*, are placed into each dish, covered and held at 27° and 80% relative humidity for 35 days. At 35 days post treatment, counts of flea adults, pupae and larvae are taken. The assay effect is expressed as the percent inhibition of adult emergence. At $3.5 \times 10^{-5}$ mg/ml or 0.0005 mg/ft$^2$ of glass surface, each of the above compounds gave 100% control of adult emergence.

### TABLE A

Compounds of formula I wherein Z is H, $CHR^1—CHR^3$ is $CH_2—CH_2$ and $R^7$ is $C_2H_5$, i.e. compounds of formula Ix

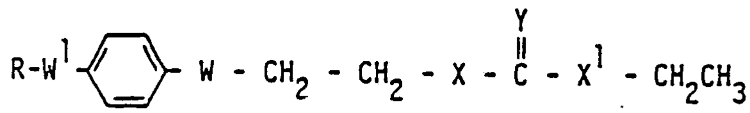

Ix

| Cpd | R | $W^1$ | W | X | Y | $X^1$ | MS m/e ($M^+$) |
|---|---|---|---|---|---|---|---|
| A-1 | $CH_3—C=CH—CH_2$<br>\|<br>$CH_3$ | O | O | NH | O | O | 293 |
| A-2 | $CH_3—CH$<br>\|<br>$CH_3$ | O | O | NH | O | O | 267 |
| A-3 | $CH≡C—CH_2$ | O | O | NH | O | O | 263 |
| A-4 | $CH≡C—CH_2—CH_2—CH_2$ | O | O | NH | O | O | 291 |
| A-5 | $Cl—CH=CH—CH_2$ | O | O | NH | O | O | 299 |
| A-6 | $CH_2=C—CH_2$<br>\|<br>$CH_3$ | O | O | NH | O | O | 279 |
| A-7 | $CH_3C=CH—CH_2$<br>\|<br>$CF_3$ | O | O | NH | O | O | 347 |
| A-8 | $CH_3—C=CH—CH_2CH_2$<br>\|<br>$CH_3$ | O | O | NH | O | O | 307 |
| A-9 | $CH_3—C=CH—CH_2$<br>\|<br>Cl | O | O | NH | O | O | 277* |
| A-10 | $CH_2=CH—CH_2—CH_2—CH_2$ | O | O | NH | O | O | 293 |
| A-11 | $CH_3—CH_2—O—CH_2$ | O | O | NH | O | O | 283 |

*$M^+$ + H

TABLE A (continued)

| Cpd | R | W[1] | W | X | Y | X[1] | MS m/e ($M^+$) |
|---|---|---|---|---|---|---|---|
| A-12 | 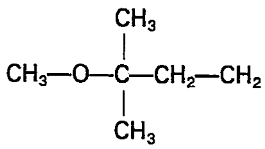 | O | O | NH | O | O | 325 |
| A-13 | 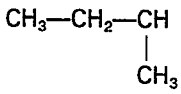 | O | O | NH | O | O | 281 |
| A-14 | 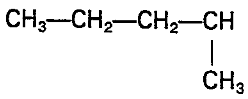 | O | O | NH | O | O | 296* |
| A-15 | $Cl—CH_2—CH_2—CH_2$ | O | O | NH | O | O | 301 |
| A-16 | 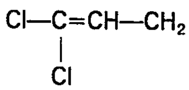 | O | O | NH | O | O | 334 |
| A-17 | 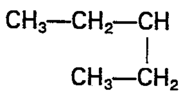 | O | O | NH | O | O | 295 |
| A-18 | 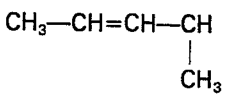 | O | O | NH | O | O | 294* |
| A-19 | 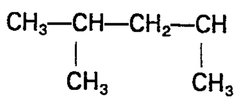 | O | O | NH | O | O | 309 |
| A-20 | 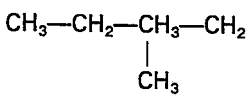 | O | O | NH | O | O | 295 |
| A-21 | 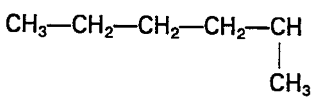 | O | O | NH | O | O | 310* |
| A-22 | $Cl—C≡C—CH_2$ | O | O | NH | O | O | 297 |
| A-23 | 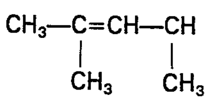 | O | O | NH | O | O | 307 |
| A-24 | 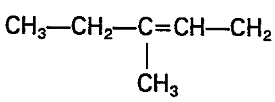 | O | O | NH | O | O | 307 |
| A-25 | 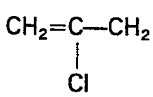 | O | O | NH | O | O | 299 |
| A-26 | 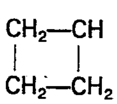 | O | O | NH | O | O | 279 |

*$M^+$ + H

TABLE A (continued)

| Cpd | R | $W^1$ | W | X | Y | $X^1$ | MS m/e ($M^+$) |
|---|---|---|---|---|---|---|---|
| A-27 | 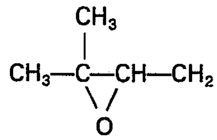 | O | O | NH | O | O | 309 |
| A-28 | $CF_3—CH_2$ | O | O | NH | O | O | 307 |
| A-29 | 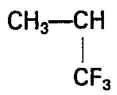 | O | O | NH | O | O | 339* |
| A-30 | 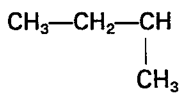 | O | O | O | O | NH | 281 |
| A-31 | 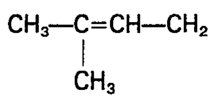 | O | O | O | O | NH | 293 |
| A-32 | 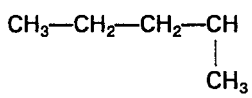 | O | O | O | O | NH | |
| A-33 | 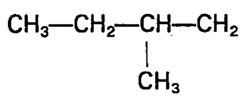 | O | O | O | O | NH | |
| A-34 | 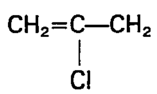 | O | O | O | O | NH | |
| A-35 | 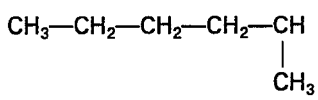 | O | O | O | O | NH | |
| A-36 | 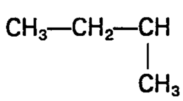 | O | O | O | S | NH | 298** |
| A-37 | 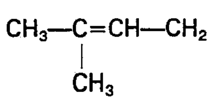 | O | O | O | S | NH | |
| A-38 | 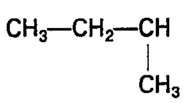 | S | O | O | S | NH | 314** |
| A-39 | 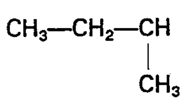 | S | O | O | O | NH | 297 |
| A-40 | 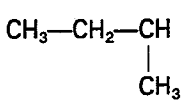 | S | O | NH | O | O | 297 |

*$M^+ + NH_4$

**$M^+ + H$

TABLE A (continued)

| Cpd | R | W[1] | W | X | Y | X[1] | MS m/e ($M^+$) |
|---|---|---|---|---|---|---|---|
| A-41 | $CH_3—C=CH—CH_2$<br>\|<br>$CH_3$ | S | O | O | S | NH | |
| A-42 | $CH_3—C=CH—CH_2$<br>\|<br>$CH_3$ | S | O | O | O | NH | 309 |
| A-43 | $CH_3—C=CH—CH_2$<br>\|<br>$CH_3$ | S | O | NH | O | O | 309 |
| A-44 | $CH_3—CH_2—CH$<br>\|<br>$CH_3$ | S | S | NH | O | O | 313 |
| A-45 | $CH_3—CH_2—CH$<br>\|<br>$CH_3$ | S | S | NH | O | S | 330* |
| A-46 | $CH_3—C=CH—CH_2$<br>\|<br>$CH_3$ | S | S | NH | O | O | |
| A-47 | $CH_3—C=CH—CH_2$<br>\|<br>$CH_3$ | S | S | NH | O | S | |
| A-48 | $CH_3—CH_2—CH$<br>\|<br>$CH_3$ | O | O | NH | O | S | 298 |
| A-49 | $CH_3—C=CH—CH_2$<br>\|<br>$CH_3$ | O | O | NH | O | S | |
| A-50 | $CH_3—CH_2—CH$<br>\|<br>$CH_3$ | S | S | NH | S | NH | 328 |
| A-51 | $CH_3—CH_2—CH$<br>\|<br>$CH_3$ | O | O | NH | S | NH | |
| A-52 | $CH_3—C=CH—CH_2$<br>\|<br>$CH_3$ | S | S | NH | S | NH | |
| A-53 | $CH_3—C=CH—CH_2$<br>\|<br>$CH_3$ | O | O | NH | S | NH | |
| A-54 | $CH_3—CH_2—CH$<br>\|<br>$CH_3$ | S | S | NH | O | NH | 313* |

*$M^+$ + H

TABLE A (continued)

| Cpd | R | $W^1$ | W | X | Y | $X^1$ | MS m/e ($M^+$) |
|---|---|---|---|---|---|---|---|
| A-55 | 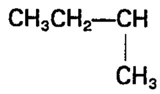 | O | O | NH | O | NH | |
| A-56 | 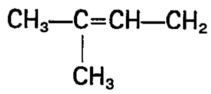 | S | S | NH | O | NH | |
| A-57 | 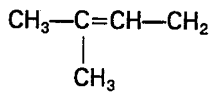 | O | O | NH | O | NH | |
| A-58 | 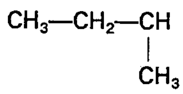 | S | S | O | O | O | 314 |
| A-59 | 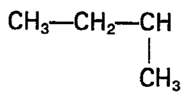 | S | O | O | O | O | 298 |
| A-60 | 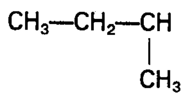 | O | O | O | O | O | 282 |
| A-61 | 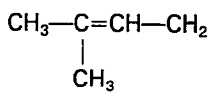 | S | S | O | O | O | |
| A-62 | 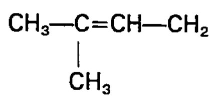 | S | O | O | O | O | |
| A-63 | 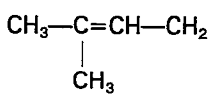 | O | O | O | O | O | |
| A-64 | 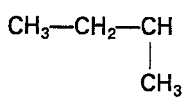 | S | S | O | O | S | 330 |
| A-65 | 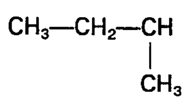 | O | O | O | O | S | 298 |
| A-66 | 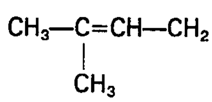 | S | S | O | O | S | |
| A-67 | 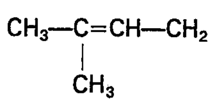 | O | O | O | O | S | |
| A-68 | 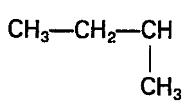 | S | S | NH | S | S | 346* |

*$M^+$ + H

TABLE A (continued)

| Cpd | R | $W^1$ | W | X | Y | $X^1$ | MS m/e ($M^+$) |
|---|---|---|---|---|---|---|---|
| A-69 | 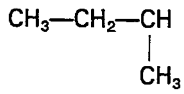 | O | O | NH | S | S | |
| A-70 | 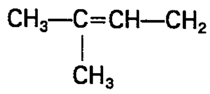 | S | S | NH | S | S | |
| A-71 | 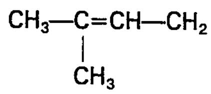 | O | O | NH | S | S | |
| A-72 | 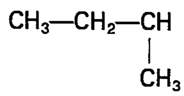 | S | S | O | S | S | 346 |
| A-73 | 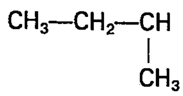 | O | O | O | S | S | 298 |
| A-74 | 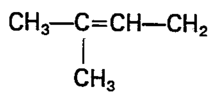 | S | S | O | S | S | |
| A-75 | 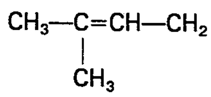 | O | O | O | S | S | |
| A-76 | 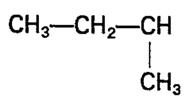 | O | S | NH | O | O | 297 |
| A-77 | 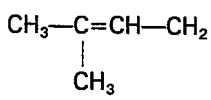 | O | S | NH | O | O | |
| A-78 | 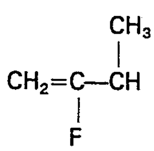 | O | O | NH | O | O | 297 |
| A-79 | 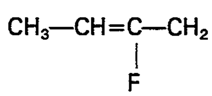 | O | O | NH | O | O | 297 |
| A-80 | 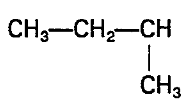 | 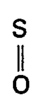 | O | NH | O | O | 314* |
| A-81 | 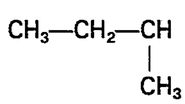 | 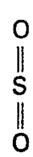 | O | NH | O | O | |

*$M^+$ + H

TABLE A (continued)

| Cpd | R | W[1] | W | X | Y | X[1] | MS m/e ($M^+$) |
|---|---|---|---|---|---|---|---|
| A-82 | $CH_3—CH_2—CH$<br>\|<br>$CH_3$ | O | O | $NCH_3$ | O | O | 295.3 |
| A-83 | $CH_3—CH_2—CH$<br>\|<br>$CH_3$ | S | O | NH | O | S | 314* |
| A-84 | $CH_3—CH_2—CH$<br>\|<br>$CH_3$ | S | O | O | O | S | 314 |
| A-85 | $CH_3—CH_2—CH$<br>\|<br>$CH_3$<br><br>\|<br>$CH_3$ | S | O | O | S | S | 330 |
| A-86 | $CH_3—CH_2—CH$<br>\|<br>$CH_3$ | S | O | NH | S | S | 330 |
| A-87 | $CH_3—CH_2—CH$<br>\|<br>$CH_3$ | O | O | S | O | NH | 298 |
| A-88 | $CH_3—CH_2—CH$<br>\|<br>$CH_3$ | S | O | S | O | NH | 314 |
| A-89 | $CH_3—C=CH—CH_2$<br>\|<br>$CH_3$ | NH | O | NH | O | O | 293* |
| A-90 | $CH_3—C=CH—CH_2$<br>\|<br>$CH_3$ | (2) | O | NH | O | O | 361* |

*$M^+$ + H
(2) $N[CH_2—CH=C(CH_3)_2]$

TABLE B

Compounds of formula I, wherein R is secondary butyl, Z is H, $R^3$ is H, compounds i.e. of formula Iy

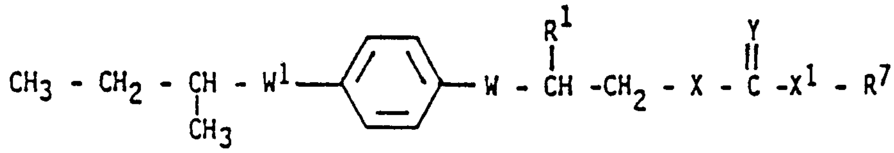
Iy

| Cpd | $W^1$ | W | $R^1$ | X | Y | $X^1$ | $R^7$ | MS m/e ($M^+$) |
|---|---|---|---|---|---|---|---|---|
| B-1 | O | O | $CH_3$ | NH | O | O | $CH_2—CH_3$ | 295 |
| B-2 | O | O | H | NH | O | O | $CH_3$ | |
| B-3 | O | O | H | NH | O | O | $CH—(CH_3)_2$ | 295 |
| B-4 | O | O | H | NH | O | O | $CH_2—CH=CH_2$ | |
| B-5 | O | O | H | NH | O | O | $CH_2—CH_2—CH_3$ | 295 |
| B-6 | O | O | H | O | O | NH | $CH—(CH_3)_2$ | 295 |
| B-7 | O | O | H | O | O | NH | $C—(CH_3)_3$ | 309 |
| B-8 | O | O | H | O | O | NH | $CH_2—CH=CH_2$ | 293 |
| B-9 | O | O | H | O | S | NH | $CH_2—CH_2—CH_3$ | 312 |
| B-10 | O | O | H | O | S | NH | $CH—(CH_3)_2$ | 311 |
| B-11 | O | O | $CH_3$ | O | O | NH | $CH_2—CH_3$ | 295 |
| B-12 | S | O | H | O | O | NH | $CH—(CH_3)_2$ | 311 |
| B-13 | S | O | $CH_3$ | O | O | NH | $CH_2—CH_3$ | 312 |
| B-16 | S | O | $CH_3$ | O | S | NH | $CH_2CH_3$ | 328 |
| B-17 | O | O | H | O | O | $N—CH_2CH_3$ | $CH_2—CH_3$ | 309 |
| B-18 | O | O | H | O | S | $N—CH_3$ | $CH_3$ | 297 |
| B-19 | O | O | h | O | S | $N—CH_2CH_3$ | $CH_2CH_3$ | 325 |
| B-20 | O | O | h | O | O | $N—CH(CH_3)_2$ | $CH(CH_3)_2$ | 338 |
| B-21 | O | O | H | O | O | NH | $CH(CH_3)—CH_2—CH_3$ | 310 |
| B-22 | O | O | H | O | O | NH | 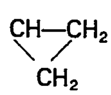 | |
| B-23 | O | O | H | NH | O | O | $CH(CH_3)_2$ | 295 |
| B-24 | O | O | H | NH | O | S | $CH(CH_3)_2$ | 312 |
| B-25 | O | O | H | NH | O | S | $CH_2CH_3$ | 298 |

TABLE B (continued)

| Cpd | $W^1$ | W | $R^1$ | X | Y | $X^1$ | $R^7$ | MS m/e ($M^+$) |
|---|---|---|---|---|---|---|---|---|
| B-26 | O | O | H | NH | O | O | $CH(CH_3)—CH_2CH_3$ | 310* |
| B-27 | O | O | $CH_3$ | NH | O | O | $CH(CH_3)_2$ | 309 |
| B-28 | O | O | $CH_3$ | NH | O | S | $CH(CH_3)_2$ | 326 |
| B-29 | O | O | $CH_3$ | NH | O | S | $CH_2—CH_3$ | 312 |
| B-30 | O | O | $CH_3$ | NH | O | O | $CH(CH_3)—CH_2—CH_3$ | |
| B-31 | S | O | $CH_3$ | NH | O | S | $CH_2—CH_3$ | 328 |
| B-32 | S | O | $CH_3$ | NH | O | O | $CH_2—CH_3$ | 311 |
| B-33 | O | O | H | NH | S | O | $CH_2—CH_3$ | 298 |
| B-34 | C=O | O | H | NH | O | O | $CH_2—CH_3$ | 293 |
| B-35 | NH | O | H | O | O | NH | $CH_2—CH_3$ | 280 |
| B-36 | O | O | H | S | O | $N—CH_3$ | $CH_3$ | 298 |
| B-37 | O | O | H | S | O | $N—CH_3$ | $CH_2CH_3$ | 312 |
| B-38 | O | O | H | O | O | NH | $C_2H_5$ | (1) |
| B-39 | O | O | H | O | O | NH | $C_2H_5$ | (2) |

*$M^+$ + H

(1) (R)-form: $[\alpha]_D^{20}$ = 19.103° (C=10% in methanol); e.g. from (S)-(+)-2-butanol via (R)-2-[4-(1-methylpropoxy)phenoxy]ethanol.

(2) (S)-form: $[\alpha]_D^{23}$ = 19.78°; e.g. from (R)-(−)-butanol via (S)-2-[4-(1-methylpropoxy)-phenoxy]ethanol.

TABLE C

Compounds of formula I, wherein $W^1$, W and Y are O, i.e. compounds of formula Iz

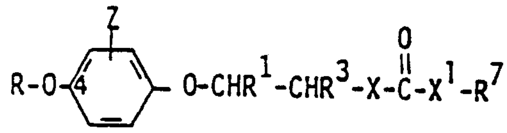

Iz

| Cpd. | R | Z | $CHR^1—CHR^3$ | X | $X^1$ | $R^7$ | MS m/e ($M^+$) |
|---|---|---|---|---|---|---|---|
| C-1 | (1) | H | $CH_2—CH(CH_3)$ | NH | O | $C_2H_5$ | 307 |
| C-2 | (2) | 2-F | $CH_2—CH_2$ | NH | O | $C_2H_5$ | |
| C-3 | (2) | $3—CH_3$ | $CH_2—CH_2$ | NH | O | $C_2H_5$ | |
| C-4 | (2) | 3—Cl | $CH_2—CH_2$ | NH | O | $C_2H_5$ | |
| C-5 | (2) | $2—CF_3$ | $CH_2—CH_2$ | NH | O | $C_2H_5$ | |
| C-6 | (1) | 2—F | $CH_2—CH_2$ | NH | O | $C_2H_5$ | |
| C-7 | (1) | $3—CH_3$ | $CH_2—CH_2$ | NH | O | $C_2H_5$ | |
| C-8 | (1) | H | $CH(CH_3)—CH_2$ | NH | O | $C_2H_5$ | 307 |
| C-9 | (1) | H | $CH_2—CH_2$ | O | NH | $i-C_3H_7$ | 307 |
| C-10 | (1) | H | $CH_2—CH_2$ | NH | O | $i-C_3H_7$ | 307 |

(1) $(CH_3)_2C=CH—CH_2$
(2) secondary butyl.

**Claims for the Contracting States: BE CH DE FR GB IT LI NL**

1. Compounds of formula I

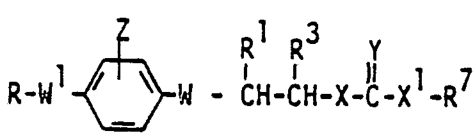

I

wherein

R is $C_{1-8}$alkyl, $C_{2-8}$alkenyl, $C_{2-8}$alkynyl, $C_{1-8}$haloalkyl, $C_{2-8}$haloalkenyl, $C_{2-8}$haloalkynyl, $C_{2-10}$-alkoxyalkyl, $C_{2-10}$alkylthioalkyl, $C_{3-8}$cycloalkyl, $C_{3-8}$halocycloalkyl, $C_{4-12}$cycloalkylalkyl, heterocycloalkyl or heterocycloalkylalkyl;

either of

$R^1$ or $R^3$ is hydrogen or $C_{1-5}$alkyl, the other is hydrogen;

$R^7$ is $C_{1-5}$alkyl, $C_{3-6}$cycloalkyl or $C_{3-5}$alkenyl;

W is oxygen or sulfur;

$W^1$ is oxygen, sulfur or carbonyl;

X and $X^1$ independently are oxygen, sulfur, NH or N($C_{1-5}$alkyl);

Y is oxygen or sulfur; and

Z is hydrogen, $C_{1-8}$alkyl, $C_{1-8}$haloalkyl or halogen.

2. A compound according to Claim 1 wherein either X is S or O and $X^1$ is NH or N($C_{1-5}$alkyl), or X is NH and $X^1$ is O or S.

3. A compound according to Claim 2 wherein X is either O or NH.

4. A compound according to Claim 3, wherein Z is H and R is $C_{1-5}$alkyl or $C_{2-5}$alkenyl.

5. A compound according to Claim 4, wherein R has 4 or 5 carbon atoms.

6. A compound according to Claim 5, wherein R is 2-methylbutyl, 2-butyl or 3-methyl-2-butenyl.

7. A method for the control of insects and acarids which comprises applying to the pest or its locus a pest controlling amount of a compound of any one of Claims 1 to 6.

8. A pest controlling composition comprising a compound of any one of Claims 1 to 6 and a diluent.

**Claims for the Contracting State: AT**

1. Pest controlling composition comprising a compound of formula I

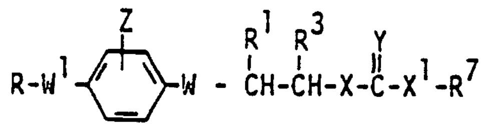

I

wherein

R is $C_{1-8}$alkyl, $C_{2-8}$alkenyl, $C_{2-8}$alkynyl, $C_{1-8}$haloalkyl, $C_{2-8}$haloalkenyl, $C_{2-8}$haloalkynyl, $C_{2-10}$-alkoxyalkyl, $C_{2-10}$alkylthioalkyl, $C_{3-8}$cycloalkyl, $C_{3-8}$halocycloalkyl, $C_{4-12}$cycloalkylalkyl, heterocycloalkyl or heterocycloalkylalkyl;

either of

$R^1$ or $R^3$ is hydrogen or $C_{1-5}$alkyl, and the other is hydrogen;

$R^7$ is $C_{1-5}$alkyl, $C_{3-6}$cycloalkyl or $C_{3-5}$alkenyl;

W is oxygen or sulfur;

$W^1$ is oxygen, sulfur or carbonyl;

X and $X^1$ independently are oxygen, sulfur, NH or N($C_{1-5}$alkyl);

Y is oxygen or sulfur; and

Z is hydrogen, $C_{1-8}$alkyl, $C_{1-8}$haloalkyl or halogen.

2. A composition according to Claim 1 wherein either X is S or O and $X^1$ is NH or N($C_{1-5}$alkyl), or X is NH and $X^1$ is O or S.

3. A composition according to Claim 2, wherein X is either O or NH.

4. A composition according to Claim 3, wherein Z is H and R is $C_{1-5}$alkyl or $C_{2-5}$alkenyl.

5. A composition according to Claim 4, wherein R has 4 or 5 carbon atoms.

6. A composition according to Claim 5, wherein R is 2-methylbutyl, 2-butyl or 3-methyl-2-butenyl.

7. A method for the control of insects and acarids which comprises applying to the pest or its locus a pest controlling amount of a compound of formula I, defined in Claims 1 to 6.

8. Process of preparing a compound of formula I stated in Claim 1, comprising

a) reacting a compound of formula II

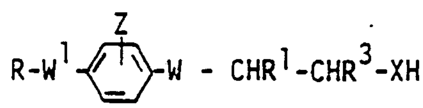

II

wherein R, $W^1$, Z, W, $R^1$, $R^3$, and X are as defined in Claim 1, with a compound of formula III

$$Q—CY—(X^1)—R^7 \qquad III$$

wherein Y, $X^1$ and $R^7$ are as defined in Claim 1, Q is halogen or

b) by reacting a compound of formula II, as defined in this claim, with a compound of formula IV

$$Y=C=NR^7 \qquad IV$$

wherein Y and $R^7$ are as defined in this Claim, or

c) by reacting a compound of formula V

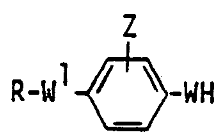

V

wherein R, $W^1$, Z and W are as defined in Claim 1, with a compound of formula VI

$$Q—CHR^1—CHR^3—X—CY—X^1—R^7 \qquad VI$$

wherein $R^1$, $R^3$, X, Y, $X^1$ and $R^7$ are as defined in Claim 1 and Q is halogen, or

d) obtaining a compound of formula Ic

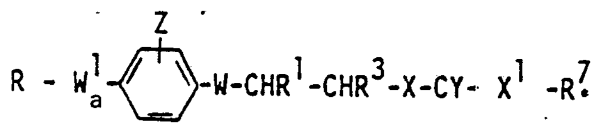

Ic

wherein $W^1_a$ is O or S and R, Z, W, $R^1$, $R^3$, X, Y, $X^1$ and $R^7$ are as defined in Claim 1, by reacting a compound of formula VII

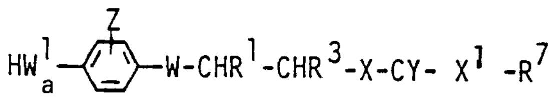

VII

wherein Z, W, $R^1$, $R^3$, X, Y, $X^1$ and $R^7$ are as defined in Claim 1, and $W^1_a$ is as defined in this claim, with a compound of formula VIII

$$RQ^1 \qquad \text{VIII}$$

wherein R is as defined in Claim 1, and $Q^1$ is halogen or mesyl, or

e) obtaining a compound of formula Id

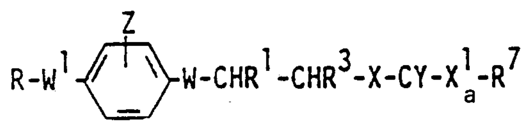

Id

wherein R, $W^1$, Z, W, $R^1$, $R^3$, X, Y and $R^7$ are as defined in Claim 1, and $X^1_a$ is O or S, by reacting a compound of formula IX

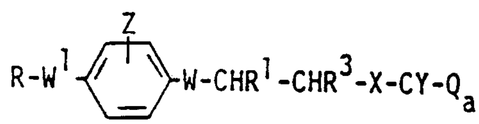

IX

wherein

$Q_a$ is OM, SM or halogen,

M is an alkali metal, and

R, $W^1$, Z, W, $R^1$, $R^3$, X and Y are as defined in Claim 1, with a compound of formula X

$$Q_bR^7 \qquad \text{X}$$

wherein $Q_b$ is either halogen where $Q_a$ is OM or SM, or is OM or SM where $Q_a$ is halogen, M being an alkali metal, and $R^7$ is as defined in Claim 1.

**Patentansprüche für die Vertragsstaaten: BE CH DE FR GB IT LI NL**

1. Verbindungen der Formel (I)

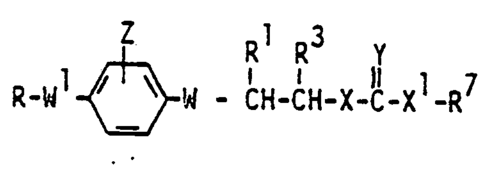

I

worin

R für $C_1$—$C_8$-Alkyl, $C_2$—$C_8$-Alkenyl, $C_2$—$C_8$-Alkinyl, $C_1$—$C_8$-Halogenalkyl, $C_2$—$C_8$-Halogenalkenyl, $C_2$—$C_8$-Halogenalkinyl, $C_2$—$C_{10}$-Alkoxyalkyl, $C_2$—$C_{10}$-Alkylthioalkyl, $C_3$—$C_8$-Cycloalkyl, $C_3$—$C_8$-Halogencycloalkyl, $C_4$—$C_{12}$-Cycloalkylalkyl, Heterocycloalkyl oder Heterocycloalkylalkyl steht,

einer der Substituenten $R^1$ oder $R^3$ Wasserstoff oder $C_1$—$C_5$-Alkyl ist und der andere Wasserstoff bedeutet,

$R^7$ für $C_1$—$C_5$-Alkyl, $C_3$—$C_6$-Cycloalkyl oder $C_3$—$C_5$-Alkenyl steht,

W Sauerstoff oder Schewefel darstellt,

$W^1$ Sauerstoff, Schwefel oder Carbonyl ist,
X und $X^1$ unabhängig Sauerstoff, Schwefel, NH oder $N(C_1—C_5$-Alkyl) sind,
Y Sauerstoff oder Schwefel ist und
Z für Wasserstoff, $C_1—C_8$-Alkyl, $C_1—C_8$-Halogenalkyl oder Halogen steht.

2. Verbindung nach Anspruch 1, dadurch gekennzeichnet, daß X für S oder O steht und $X^1$ für NH oder $N(C_1—C_5$-Alkyl) steht oder daß X für NH steht und $X^1$ für O oder S steht.

3. Verbindung nach Anspruch 2, dadurch gekennzeichnet, daß X entweder O oder NH ist.

4. Verbindung nach Anspruch 3, dadurch gekennzeichnet, daß Z für H steht und R für $C_1—C_5$-Alkyl oder $C_2—C_5$-Alkenyl steht.

5. Verbindung nach Anspruch 4, dadurch gekennzeichnet, daß R 4 oder 5 Kohlenstoffatome aufweist.

6. Verbindung nach Anspruch 5, dadurch gekennzeichnet, daß R für 2-Methylbutyl, 2-Butyl oder 3-Methyl-2-butenyl steht.

7. Verfahren zur Bekämpfung von Insekten und Akariden, dadurch gekennzeichnet, daß die Schädlinge oder ihr Lebensraum mit einer schädlingsbekämpfenden Menge einer Verbindung nach einem der Ansprüche 1 bis 6 behandelt werden.

8. Schädlingsbekämpfungsmittel, dadurch gekennzeichnet, daß es eine Verbindung nach einem der Ansprüche 1 bis 6 als Wirkstoff und ein Verdünnungsmittel enthält.

**Patentansprüche für den Vertragsstaat: AT**

1. Schädlingsbekämpfungsmittel, dadurch gekennzeichnet, daß es eine Verbindung der Formel (I)

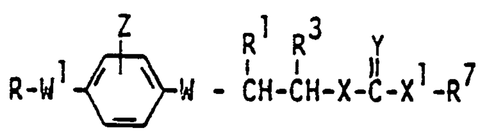

I

worin
R für $C_1—C_8$-Alkyl, $C_2—C_8$-Alkenyl, $C_2—C_8$-Alkinyl, $C_1—C_8$-Halogenalkyl, $C_2—C_8$-Halogenalkenyl, $C_2—C_8$-Halogenalkinyl, $C_2—C_{10}$-Alkoxyalkyl, $C_2—C_{10}$-Alkylthioalkyl, $C_3—C_8$-Cycloalkyl, $C_3—C_8$-Halogencycloalkyl, $C_4—C_{12}$-Cycloalkylalkyl, Heterocycloalkyl oder Heterocycloalkylalkyl steht,
einer der Substituenten $R^1$ oder $R^3$ Wasserstoff oder $C_1—C_5$-Alkyl ist und der andere Wasserstoff bedeutet,
$R^7$ für $C_1—C_5$-Alkyl, $C_3—C_6$-Cycloalkyl oder $C_3—C_5$-Alkenyl steht,
W Sauerstoff oder Schewefel darstellt,
$W^1$ Sauerstoff, Schwefel oder Carbonyl ist,
X und $X^1$ unabhängig Sauerstoff, Schwefel, NH oder $N(C_1—C_5$-Alkyl) sind,
Y Sauerstoff oder Schwefel ist und
Z für Wasserstoff, $C_1—C_8$-Alkyl, $C_1—C_8$-Halogenalkyl oder Halogen steht, als Wirkstoff enthält.

2. Mittel nach Anspruch 1, dadurch gekennzeichnet, daß X für S oder O steht und $X^1$ für NH oder $N(C_1—C_5$-Alkyl) steht oder daß X für NH steht und $X^1$ für O oder S steht.

3. Mittel nach Anspruch 2, dadurch gekennzeichnet, daß X entweder O oder NH ist.

4. Mittel nach Anspruch 3, dadurch gekennzeichnet, daß Z für H steht und R für $C_1—C_5$-Alkyl oder $C_2—C_5$-Alkenyl steht.

5. Mittel nach Anspruch 4, dadurch gekennzeichnet, daß R 4 oder 5 Kohlenstoffatome aufweist.

6. Mittel nach Anspruch 5, dadurch gekennzeichnet, daß R für 2-Methylbutyl, 2-Butyl oder 3-Methyl-2-butenyl steht.

7. Verfahren zur Bekämpfung von Insekten und Akariden, dadurch gekennzeichnet, daß die Schädlinge oder ihr Lebensraum mit einer schädlingsbekämpfenden Menge einer Verbindung der Formel (I) nach einem der Ansprüche 1 bis 6 behandelt werden.

8. Verfahren zur Herstellung einer Verbindung der formel (I) gemäß Anspruch 1, dadurch gekennzeichnet, daß

(a) eine Verbindung der Formel (II)

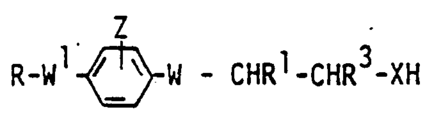

II

worin R, $W^1$, Z, W, $R^1$, $R^3$ und X wie in Anspruch 1 definiert sind, mit einer Verbindung der Formel (III)

$$Q—CY—(X^1)—R^7 \qquad (III)$$

worin Y, $X^1$ und $R^7$ wie in Anspruch 1 definiert sind und Q Halogen ist, umgesetzt wird, oder

(b) eine Verbindung der Formel (II) gemäß Definition in diesem Anspruch mit einer Verbindung der Formel (IV)

$$Y{=}C{=}NR^7 \qquad (IV)$$

worin Y und $R^7$ wie in diesem Anspruch definiert sind, umgesetzt wird, oder

(c) eine Verbindung der Formel (V)

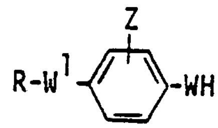

V

worin R, $W^1$, Z und W wie in Anspruch 1 definiert sind, mit einer Verbindung der Formel (VI)

$$Q{-}CHR^1{-}CHR^3{-}X{-}CY{-}X^1{-}R^7 \qquad (VI)$$

worin $R^1$, $R^3$, X, Y, $X^1$ und $R^7$ wie in Anspruch 1 definiert sind und Q Halogen ist, umgesetzt wird, oder

(d) zur Herstellung einer Verbindung der Formel (Ic)

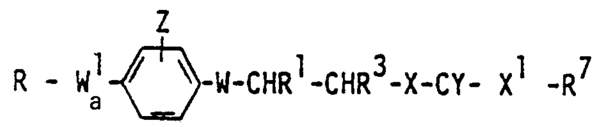

Ic

worin $W_a^1$ O oder S ist und R, Z, W, $R^1$, $R^3$, X, Y, $X^1$ und $R^7$ wie in Anspruch 1 definiert sind, eine Verbindung der Formel (VII)

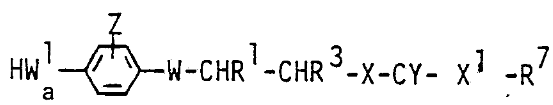

VII

worin Z, W, $R^1$, $R^3$, X, Y, $X^1$ und $R^7$ wie in Anspruch 1 definiert sind und $W_a^1$ wie in diesem Anspruch definiert ist, mit einer Verbindung der Formel (VIII)

$$RQ^1 \qquad (VIII)$$

worin R wie in Anspruch 1 definiert ist und $Q^1$ Halogen oder Mesyl bedeutet, umgesetzt wird, oder

(e) zur Herstellung einer Verbindung der Formel (Id)

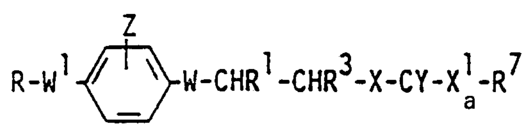

Id

worin R, $W^1$, Z, W, $R^1$, $R^3$, X, Y und $R^7$ wie in Anspruch 1 definiert sind und $X_a^1$ O oder S ist, eine Verbindung der Formel (IX)

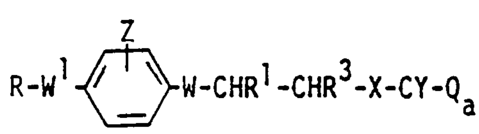

IX

worin $Q_a$ für OM, SM oder Halogen steht, M ein Alkalimetall ist und R, $W^1$, Z, W, $R^1$, $R^3$, X und Y wie in Anspruch 1 definiert sind, mit einer Verbindung der Formel (X)

$$Q_bR^7 \qquad (X)$$

worin $Q_b$ entweder Halogen bedeutet, falls $Q_a$ für OM oder SM steht, oder OM oder SM bedeutet, falls $Q_a$ Halogen ist, M ein Alkalimetall ist und $R^7$ wie in Anspruch 1 definiert ist, umgesetzt wird.

**Revendications pour les Etats contractants: BE CH DE FR GB IT LI NL**

1. Les composés de formule I

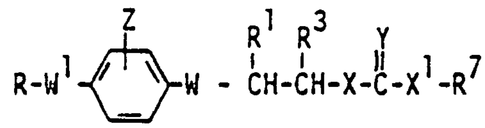

I

dans laquelle:

R signifie un groupe alkyle en $C_1$—$C_8$, alcényle en $C_2$—$C_8$, alcynyle en $C_2$—$C_8$, haloalkyle en $C_1$—$C_8$, haloalcényle en $C_2$—$C_8$, haloalcynyle en $C_2$—$C_8$, alcoxyalkyle en $C_2$—$C_{10}$, alkylthioalkyle en $C_2$—$C_{10}$, cycloalkyle en $C_3$—$C_8$, halocycloalkyle en $C_3$—$C_8$, cycloalkylalkyle en $C_4$—$C_{12}$, hétérocycloalkyle ou hétérocycloalkylalkyle, l'un de $R^1$ ou $R^3$ signifie l'hydrogène ou un groupe alkyle en $C_1$—$C_5$ et l'autre signifie l'hydrogène,

$R^7$ signifie un groupe alkyle en $C_1$—$C_5$, cycloalkyle en $C_3$—$C_6$ ou alcényle en $C_3$—$C_5$,

W signifie l'oxygène ou le soufre,

$W^1$ signifie l'oxygène, le soufre ou un groupe carbonyle,

X et $X^1$ signifient indépendamment l'oxygène, le soufre, NH ou N(alkyle en $C_1$—$C_5$),

Y signifie l'oxygène ou le soufre et

Z signifie l'hydrogène, un groupe alkyle en $C_1$—$C_8$ ou haloalkyle en $c_1$—$C_8$ ou un halogène.

2. Un composé selon la revendication 1, dans lequel X signifie S ou O et $X^1$ signifie NH ou N(alkyle en $C_1$—$C_5$), ou bien X signifie NH et $X^1$ signifie O ou S.

3. Un composé selon la revendication 2, dans lequel X signifie O ou NH.

4. Un composé selon la revendication 3, dans lequel Z signifie H et R signifie un groupe alkyle en $C_1$—$C_5$ ou alcényle en $C_2$—$C_5$.

5. Un composé selon la revendication 4, dans lequel R contient 4 ou 5 atomes de carbone.

6. Un composé selon la revendication 5, dans lequel R signifie un groupe 2-méthylbutyle, 2-butyle ou 3-méthyl-2-butényle.

7. Une méthode de lutte contre les insectes et les acariens, qui comprend l'application sur l'organisme nuisible ou son habitat, d'une quantité d'un composé selon l'une quelconque des revendications 1 à 6 combattant l'organisme nuisible.

8. Une composition destinée à combattre les organismes nuisibles, comprenant un composé selon l'une quelconque des revendications 1 à 6 et un diluant.

**Revendications pour l'Etat contractant: AT**

1. Une composition destinée à combattre les organismes nuisibles, comprenant un composé de formule I

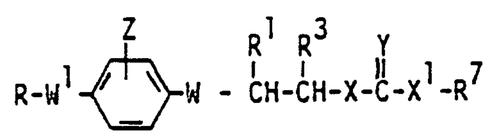

I

dans laquelle:

R signifie un groupe alkyle en $C_1$—$C_8$, alcényle en $C_2$—$C_8$, alcynyle en $C_2$—$C_8$, haloalkyle en $C_1$—$C_8$, haloalcényle en $C_2$—$C_8$, haloalcynyle en $C_2$—$C_8$, alcoxyalkyle en $C_2$—$C_{10}$, alkylthioalkyle en $C_2$—$C_{10}$, cycloalkyle en $C_3$—$C_8$, halocycloalkyle en $C_3$—$C_8$, cycloalkylalkyle en $C_4$—$C_{12}$, hétérocycloalkyle ou hétérocycloalkylalkyle, l'un de $R^1$ ou $R^3$ signifie l'hydrogène ou un groupe alkyle en $C_1$—$C_5$ et l'autre signifie l'hydrogène,

$R^7$ signifie un groupe alkyle en $C_1$—$C_5$, cycloalkyle en $C_3$—$C_6$ ou alcényle en $C_3$—$C_5$,

W signifie l'oxygène ou le soufre,

$W^1$ signifie l'oxygène, le soufre ou un groupe carbonyle,

X et $X^1$ signifient indépendamment l'oxygène, le soufre, NH ou N(alkyle en $C_1$—$C_5$),

Y signifie l'oxygène ou le soufre et

Z signifie l'hydrogène, un groupe alkyle en $C_1$—$C_8$ ou haloalkyle en $C_1$—$C_8$ ou un halogène.

2. Une composition selon la revendication 1, dans laquelle X signifie S ou O et $X^1$ signifie NH ou N(alkyle en $C_1$—$C_5$), ou bien X signifie NH et $X^1$ signifie O ou S.

3. Une composition selon la revendication 2, dans laquelle X signifie O ou NH.

4. Une composition selon la revendication 3, dans laquelle Z signifie H et R signifie un groupe alkyle en $C_1$—$C_5$ ou alcényle en $C_2$—$C_5$.

5. Une composition selon la revendication 4, dans laquelle contient 4 ou 5 atomes de carbone.

6. Une composition selon la revendication 5, dans laquelle R signifie un groupe 2-méthylbutyle, 2-butyle ou 3-méthyl-2-buténylé.

7. Une méthode de lutte contre les insectes et les acariens, qui comprend l'application sur l'organisme nuisible ou son habitat, d'une quantité d'un composé de formule I tel que défini aux revendications 1 à 6 combattant l'organisme nuisible.

8. Un procédé de préparation d'un composé de formule I tel que spécifié à la revendication 1, comprenant

a) la réaction d'un composé de formule II

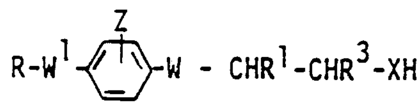 II

dans laquelle

R, $W^1$, Z, W, $R^1$, $R^3$ et X sont tels que définis à la revendication 1, avec un composé de formule III

$$Q—CY—(X^1)—R^7 \qquad III$$

dans laquelle Y, $X^1$ et $R^7$ sont tels que définis à la revendication 1, Q signifie un halogène, ou

b) la réaction d'un composé de formule II, tel que défini à cette revendication, avec un composé de formule IV

$$Y=C=NR^7 \qquad IV$$

dans laquelle Y et $R^7$ sont tels que définis à cette revendication, ou

c) la réaction d'un composé de formule V

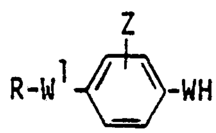 V

dans laquelle R, $W^1$, Z et W sont tels que définis à la revendication 1, avec un composé de formule VI

$$Q—CHR^1—CHR^3—X—CY—(X^1)—R^7 \qquad VI$$

dans laquelle $R^1$, $R^2$, X, Y, $X^1$ et $R^7$ sont tels que définis à la revendication 1 et Q signifie un halogène, ou

d) l'obtention d'un composé de formule Ic

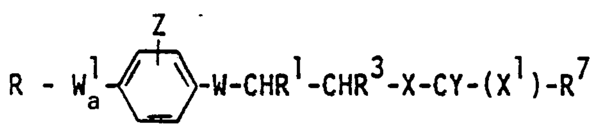 Ic

dans laquelle $W^1_a$ signifie O ou S et R, Z, W, $R^1$, $R^3$, X, Y, $X^1$ et $R^7$ sont tels que définis à la revendication 1, en faisant réagir un composé de formule VII

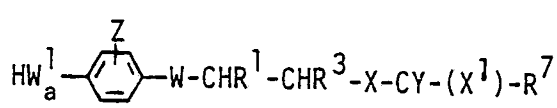 VII

dans laquelle Z, W, $R^1$, $R^3$, X, Y, $X^1$ et $R^7$ sont tels que définis à la revendication 1, et $W^1_a$ est tel que défini à cette revendication, avec un composé de formule VIII

$$RQ^1 \qquad VIII$$

dans laquelle R est tel que défini à la revendication 1, et $Q^1$ signifie un halogène ou un groupe mésyle, ou

e) l'obtention d'un composé de formule Id

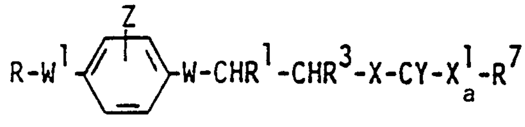 Id

dans laquelle

R, $W^1$, Z, W, $R^1$, $R^3$, X, Y, et $R^7$ sont tels que définis à la revendication 1, et $X^1_a$ signifie O ou S, en faisant réagir un composé de formule IX

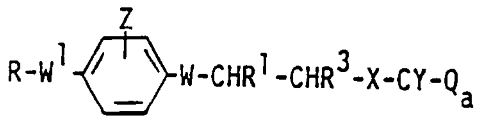

IX

dans laquelle

$Q_a$ signifie OM, SM ou un halogène,

M signifie un métal alcalin, et

R, $W^1$, Z, W, $R^1$, $R^3$, X et Y sont tels que définis à la revendication 1, avec un composé de formule X

$$Q_bR^7 \qquad X$$

dans laquelle $Q_b$ signifie un halogène lorsque $Q_a$ signifie OM ou SM, ou bien signifie OM ou SM lorsque $Q_a$ signifie un halogène, M étant un métal alcalin, et $R^7$ est tel que défini à la revendication 1.